# EUROPEAN PATENT APPLICATION

(11) **EP 3 878 478 A1**
(43) Date of publication of application: **15.09.2021**
(21) Application number: 19881263.8
(22) Date of filing: 01.11.2019
(51) Int. Cl.: A61L 9/20, B01D 53/44, B01D 53/76, B08B 3/02

(54) **GAS TREATMENT DEVICE**

(30) Priority: 06.11.2018 JP 2018209246
(71) Applicant: Ushio Denki Kabushiki Kaisha, Tokyo 100-8150 (JP)
(72) Inventor: NAITO,Keisuke, Tokyo 100-8150 (JP)
(74) Representative: Tomerius, Isabel
(86) International application number: PCT/JP2019/043009
(87) International publication number: WO 2020/095835

(57) **Abstract**

Provided is a gas treatment device that can prevent a decrease in capacity for treatment of a VOC-containing gas to be treated over time.

A gas treatment device includes: a housing; a gas inlet to introduce a gas to be treated containing VOC into the housing; an excimer lamp housed inside the housing, the excimer lamp including a tube body with a long shape extending in a first direction, an interior of the tube body being filled with a discharge gas; a gas outlet to exhaust the gas to be treated to outside the housing, the gas to be treated being irradiated with ultraviolet light emitted from the excimer lamp; and a cleaning mechanism to spray a first fluid from an inside of the housing onto a surface of the tube body, the first fluid containing moisture and being used for cleaning.

## Description

### TECHNICAL FIELD

The present invention relates to a gas treatment device. In particular, the invention relates to a device for treating a gas to be treated using an excimer lamp.

### BACKGROUND ART

Conventionally, techniques have been presented to purify gases to be treated using low-pressure mercury lamps. For instance, Patent Document 1 described below discloses that a low-pressure mercury lamp that emits ultraviolet light having a wavelength of 185 nm or a wavelength of 254 nm is used to decompose and remove impurities and bacteria contained in a gas to be treated. Specifically, a description is given of a technique for generating an ozone (03) gas by ultraviolet light having a wavelength of 185 nm and decomposing impurities and a malodorous substance by the ozone gas.

Patent Document 2 described below discloses a xenon excimer lamp designed to emit light at 172 nm, which is a wavelength shorter than the wavelengths of the above low-pressure mercury lamp.

### PRIOR ART DOCUMENT

### PATENT DOCUMENTS

Patent Document 1: JP-A-2006-204683
Patent Document 2: JP-A-2007-335350

### SUMMARY OF THE INVENTION

### PROBLEMS TO BE SOLVED BY THE INVENTION

Conventional excimer lamps, as is disclosed in Patent Document 2, have been used to remove organic substances in manufacturing processes for semiconductors or liquid crystal panels. In other words, excimer lamps have been normally used in a clean environment that is precisely controlled.

The inventor has been examining a way of increasing efficiency in decomposition of volatile organic compounds (VOC) and improving sterilization performance by using this excimer lamp instead of the low-pressure mercury lamp as is described in Patent Document 1 and irradiating a gas to be treated with light having a shortened wavelength. In particular, when the gas to be treated contains oxygen and moisture and the gas is irradiated with short-wavelength light emitted from the excimer lamp, O(¹D) and a hydroxy radical (•OH), which are highly reactive, are generated. Thus, high performance in decomposition of VOC contained in the gas to be treated is anticipated.

The inventor has extensively studied on treatment of a VOC-containing gas to be treated using an excimer lamp and found a new problem of this technique. As the treatment continues, a contaminant gets deposited on a surface of a tube body of the excimer lamp and an illuminance of ultraviolet light emitted from the excimer lamp decreases. An occurrence of such a situation results in a decrease in capacity for treatment of VOC over time and thus is unfavorable.

The inventor conducted an experiment that involved putting off an excimer lamp and letting a VOC-containing gas to be treated pass through an immediate vicinity of the unlit excimer lamp. As a result, even after a lapse of time, contamination was not identified on a tube body of the excimer lamp. Based on this result, the inventor has presumed that irradiating a VOC-containing gas to be treated with ultraviolet light emitted from an excimer lamp that is lit causes generation of the contaminant and deposition of the contaminant on the tube body.

It is an object of the present invention to provide a gas treatment device that can prevent a decrease in capacity for treatment of a VOC-containing gas to be treated over time.

### MEANS FOR SOLVING THE PROBLEMS

A gas treatment device according to an aspect of the present invention includes:
a housing;
a gas inlet to introduce a gas to be treated containing volatile organic compounds (VOC) into the housing;
an excimer lamp housed inside the housing, the excimer lamp including a tube body with a long shape extending in a first direction, an interior of the tube body being filled with a discharge gas;
a gas outlet to exhaust the gas to be treated to outside the housing, the gas to be treated being irradiated with ultraviolet light emitted from the excimer lamp; and
a cleaning mechanism to spray a first fluid from an inside of the housing onto a surface of the tube body, the first fluid containing moisture and being used for cleaning.

Owing to the extensive study on treatment of the VOC-containing gas to be treated using the lit excimer lamp, the inventor has found that the contaminant deposited on the surface of the tube body of the excimer lamp as a result of continuation of the treatment can be dissolved in a fluid containing moisture and come off the surface of the tube body. It can be inferred that the contaminant is a substance derived from VOC and is a secondary product generated by being irradiated with ultraviolet light. According to the configuration, the gas treatment device includes the cleaning mechanism to spray the first fluid containing moisture. Thus, even if a contaminant gets deposited on the surface of the tube body, the gas treatment device allows the contaminant to come off the surface of the tube body by spraying the first fluid onto the surface of the tube body. This can prevent a decrease in illuminance of the ultraviolet light over time.

The excimer lamp may include a base made of an inorganic material at an end of the tube body in the first direction, and the cleaning mechanism may spray the fluid for cleaning onto the surface of the tube body positioned inward from the base.

The base is a member having a function of supporting the end of the tube body and is made of, for example, a ceramic material such as steatite, forsterite, sialon, or alumina. Power feeders are placed through a hole formed in the base or along an outer edge of the base to supply a voltage to the excimer lamp. Thus, if a fluid containing moisture is sprayed onto the base and the base absorbs the moisture, the absorbed moisture creates a risk of a short circuit between the power feeders placed through the base. According to the above configuration, the cleaning mechanism sprays the first fluid onto the surface of the tube body positioned inward from the base. This reduces the risk that the moisture is absorbed by the base when a cleaning process is performed.

The housing may include a drainage outlet to drain a second fluid in liquid form to outside the housing. The second fluid is left after the surface of the tube body is cleaned with the first fluid.

This configuration allows the second fluid, which is produced when the VOC-derived secondary product is dissolved in the moisture, to be drained to outside the housing through the drainage outlet. This reduces the occurrence of a problem of accumulation of the second fluid inside the housing.

The cleaning mechanism may include:
a water injection pipe disposed so as to pass through a part of the housing; and
a water supply pipe disposed outside the housing to supply water as the first fluid to the water injection pipe, and
the water injection pipe may spray the water as the first fluid onto the surface of the tube body.

The cleaning mechanism may allow a target onto which the first fluid is sprayed to be shifted along the first direction.

According to this configuration, a contaminant deposited over a wide area of the surface of the tube body of the excimer lamp can come off.

The cleaning mechanism may include:
a nozzle member that has a ring shape so as to substantially surround an outer periphery of the surface of the tube body and that has a plurality of holes in a surface facing the tube body; and
a water supply pipe to supply water as the first fluid to the nozzle member.

According to this configuration, a contaminant deposited circumferentially over a wide area of the surface of the tube body can come off.

The cleaning mechanism may include an air knife to spray humidified air as the first fluid in planar form.

According to this configuration, a distal end of the air knife is directed at the surface of the tube body. This allows the contaminant to readily come off the surface of the tube body by a wind current from the air knife while allowing the contaminant to be dissolved in the moisture.

The excimer lamp included in the gas treatment device may have any shape.

In one example, the excimer lamp may include a first electrode disposed at a predetermined place on an outer surface of the tube body and a second electrode that is disposed on an outer surface of the tube body and at a place opposite to the first electrode through the interior of the tube body filled with the discharge gas. In this case, the tube body may have two faces that are substantially flat in shape and opposite to each other, and the first electrode may be disposed on one of the faces and the second electrode may be disposed on the other face.

In another example, the excimer lamp may include a first electrode disposed at a predetermined place on an outer surface of the tube body and a second electrode disposed in the interior of the tube body filled with the discharge gas.

In still another example, the excimer lamp may have a double-tube structure such that the tube body includes an outside tube and an inside tube disposed inside the outside tube and such that the outside tube and the inside tube are sealed off at both ends of the tube body in the first direction,
a space between the inside tube and the outside tube may be filled with the discharge gas, and
the excimer lamp may include a first electrode disposed on an outer surface of the outside tube and a second electrode disposed on an inner surface of the inside tube.

### EFFECT OF THE INVENTION

A gas treatment device according to the present invention can prevent a decrease in capacity for treatment of a VOC-containing gas to be treated over time.

### BRIEF DESCRIPTION OF THE DRAWINGS

Fig. 1 is a schematic cross-sectional view showing a configuration of a gas treatment device according to an embodiment of the present invention.
Fig. 2 is a schematic plan view showing an excimer lamp included in the gas treatment device of Fig. 1 together with a cleaning mechanism, viewed along a direction d1.
Fig. 3 is a schematic cross-sectional view showing a configuration of a gas treatment device according to an embodiment of the present invention.
Fig. 4 is a schematic plan view showing an excimer lamp included in the gas treatment device of Fig. 3 together with a cleaning mechanism, viewed along a direction d1.
Fig. 5 is a schematic cross-sectional view showing a configuration of a gas treatment device according to another embodiment of the present invention.
Fig. 6 is a schematic plan view showing an excimer lamp included in the gas treatment device of Fig. 5 together with a cleaning mechanism, viewed along a direction d1.
Fig. 7 is a schematic cross-sectional view showing a configuration of a gas treatment device according to another embodiment of the present invention.
Fig. 8 is a schematic plan view of an excimer lamp that has another example configuration and that is included in a gas treatment device of the present invention, viewed along a direction d1.
Fig. 9 is a schematic plan view of an excimer lamp that has still another example configuration and that is included in a gas treatment device of the present invention, viewed along a direction d1.

### MODE FOR CARRYING OUT THE INVENTION

Gas treatment devices according to embodiments of the present invention will be described with reference to the drawings as appropriate. All the drawings shown below are schematic views, and dimensions or proportions in the drawings do not always coincide with actual dimensions or proportions. Dimensions or proportions do not always coincide between the drawings.

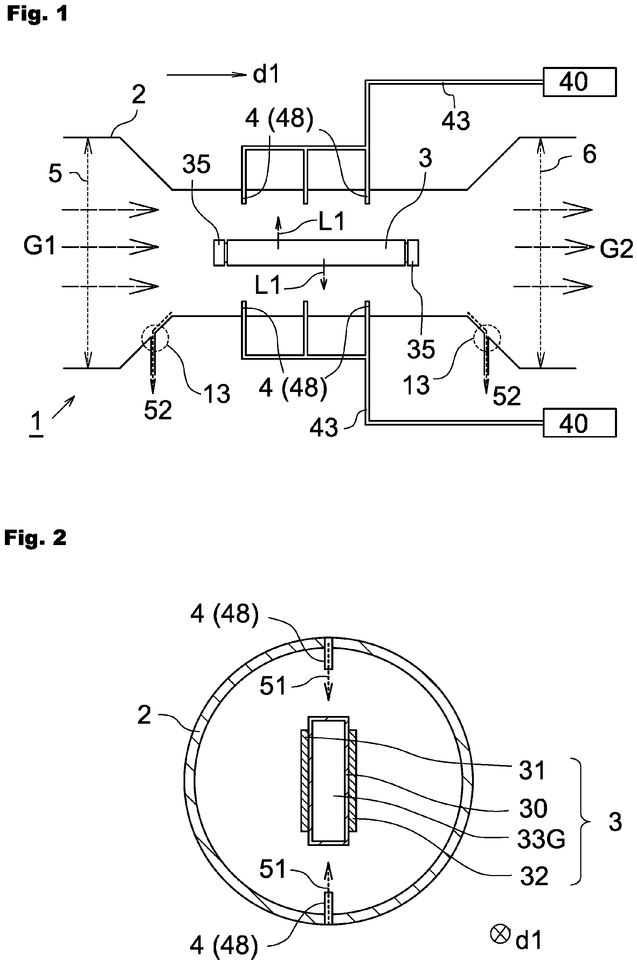

Fig. 1 is a schematic cross-sectional view showing a configuration of a gas treatment device according to an embodiment. A gas treatment device 1 shown in Fig. 1 includes a housing 2, an excimer lamp 3 housed inside the housing 2, and a cleaning mechanism 4.

The excimer lamp 3 extends along a direction d1 (corresponding to a "first direction") in shape. Fig. 2 is a schematic plan view showing the excimer lamp 3 together with the cleaning mechanism 4, viewed along the direction d1. In the present embodiment, the excimer lamp 3 includes a tube body 30 extending along the direction d1, a first electrode 31 disposed on an outer surface of the tube body 30, and a second electrode 32 that is disposed on an outer surface of the tube body 30 and at a place opposite to the first electrode 31. The tube body 30 is made of a dielectric substance such as synthetic fused silica. An interior of the tube body 30 is filled with a discharge gas 33G that forms excimer molecules by electrical discharging. In the present embodiment, the discharge gas 33G contains xenon (Xe). In one specific example, the discharge gas 33G is a gas that contains both xenon (Xe) and neon (Ne) in a predetermined ratio (e.g., 3:7) and may further contain oxygen and hydrogen in minute quantities.

An alternating-current voltage at a high frequency, for example, approximately from 50 kHz to 5 MHz is applied between the first electrode 31 and the second electrode 32, and the voltage is thereby applied to the discharge gas 33G via the tube body 30 to produce discharge plasma in a discharge space filled with the discharge gas 33G. As a result, atoms in the discharge gas 33G are excited and get into an excimer state. When the atoms transition to a ground state, excimer light is generated. When the discharge gas 33G is the gas that contains xenon (Xe) described above, the excimer light is ultraviolet light L1 having a wavelength close to 172 nm. The discharge gas 33G may contain a different substance to change the wavelength of the ultraviolet light L1. For instance, the discharge gas 33G may be a gas that contains a substance such as ArBr (165 nm), ArCI (175 nm), or F2 (153 nm).

The first electrode 31 and the second electrode 32 each have a mesh shape (a net shape) or a linear shape so as not to hinder the ultraviolet light L1 generated inside the tube body 30 from being emitted to outside the tube body 30.

The gas treatment device 1 is used to treat a gas to be treated G1 containing volatile organic compounds (VOC). The VOC is a general term for organic compounds that have volatility and that are in gaseous form in the atmosphere, and is a general term for a group of substances including formaldehyde, toluene, xylene, acetone, and the like. It is assumed that the gas treatment device 1 is used, for example, by being directly connected with an air-conditioning duct in a building or an exhaust gas duct in a plant.

As shown in Fig. 1, the gas treatment device 1 has a gas inlet 5 to introduce the gas to be treated G1 containing the VOC into the housing 2 and a gas outlet 6 to exhaust a gas (a treated gas G2) to outside the housing 2 after the gas to be treated G1 is irradiated with the ultraviolet light L1 from the excimer lamp 3.

The gas to be treated G1 containing the VOC and being suctioned from the gas inlet 5 is irradiated with the ultraviolet light L1 emitted from the excimer lamp 3 and thereby generates O(¹D) and a hydroxy radical (•OH), which are highly reactive, through reactions shown in the following formulas (1) and (2). In the following formulas, O(¹D) represents an oxygen atom in an excited state, and O(³P) represents an oxygen atom in a ground state. In addition, hν(λ) represents absorption of light at a wavelength λ.

O₂ + hν(λ) → O(¹D) + O(³P) ... (1)

O(¹D) + H₂O → •OH + •OH ... (2)

The reaction shown in the above formula (1) is apt to occur when the wavelength λ of the ultraviolet light L1 is less than or equal to 180 nm.

The reactivity of O(¹D) and the hydroxy radical (•OH) is higher than that of ozone (O₃). Thus, as compared to the conventional gas treatment device designed to decompose a gas by ozone generated using the low-pressure mercury lamp, the gas treatment device 1 can efficiently decompose the gas to be treated G1 containing a VOC-derived substance such as formaldehyde, which is hard to decompose by ozone.

The gas treatment device 1 includes the cleaning mechanism 4. In the present embodiment, the cleaning mechanism 4, as shown in Fig. 2, has a plurality of water injection pipes 48. Each of the water injection pipes 48 is, as shown in Fig. 1, connected to a water supply source 40 through a water supply pipe 43. The water injection pipes 48 are formed so as to pass through some places of the housing 2 and are designed to spray water onto the tube body 30. The water is stored as a first fluid 51 in the water supply sources 40 disposed outside the housing 2.

In illustrations of Figs. 1 and 2, the water injection pipes 48 are formed only above and below the tube body 30 when viewed along the direction d1. However, the water injection pipes 48 may be disposed in any form. In other words, the water injection pipes 48 may be disposed at a plurality of places such that water as the first fluid 51 is allowed to be sprayed onto a surface of the tube body 30 in a plurality of directions.

Even if a secondary product derived from the VOC is deposited on the surface of the tube body 30, the above configuration allows the secondary product to be dissolved in the water supplied from the cleaning mechanism 4 (the water injection pipes 48). This can clean the surface of the tube body 30 and prevent a decrease in illuminance of the ultraviolet light L1. This feature will be described later with reference to examples.

In the present embodiment, the gas treatment device 1 includes a drainage outlet 13. Since the water (the first fluid 51) is sprayed onto the surface of the tube body 30, the VOC-derived secondary product deposited on the surface of the tube body 30 is dissolved in the water and as a result, a fluid (a second fluid 52) is produced. The drainage outlet 13 is used to drain the second fluid to outside the housing 2. The drainage outlet 13 may have any shape and may be disposed at any place, with the proviso that the drainage outlet is configured to drain the second fluid 52, which is produced inside the housing 2, to outside the housing 2.

The excimer lamp 3 includes a base 35 at each end of the tube body 30 in the direction d1. The bases 35 are made of a ceramic material (an inorganic material) such as steatite, forsterite, sialon, or alumina and have a function of fixing the ends of the tube body 30.

Power feeders (not shown) are placed through a hole formed in the bases 35 or along an outer edge of the bases 35 to supply electricity to the first electrode 31 and the second electrode 32. Thus, if the base 35 absorbs moisture, the absorbed moisture creates a risk of a short circuit between the power feeders placed in close vicinity of the base 35. In view of avoiding such a short-circuit fault, it is preferred that the cleaning mechanism 4 be configured not to spray water onto the bases 35 but to spray water onto the surface of the tube body 30 positioned inward from the bases 35.

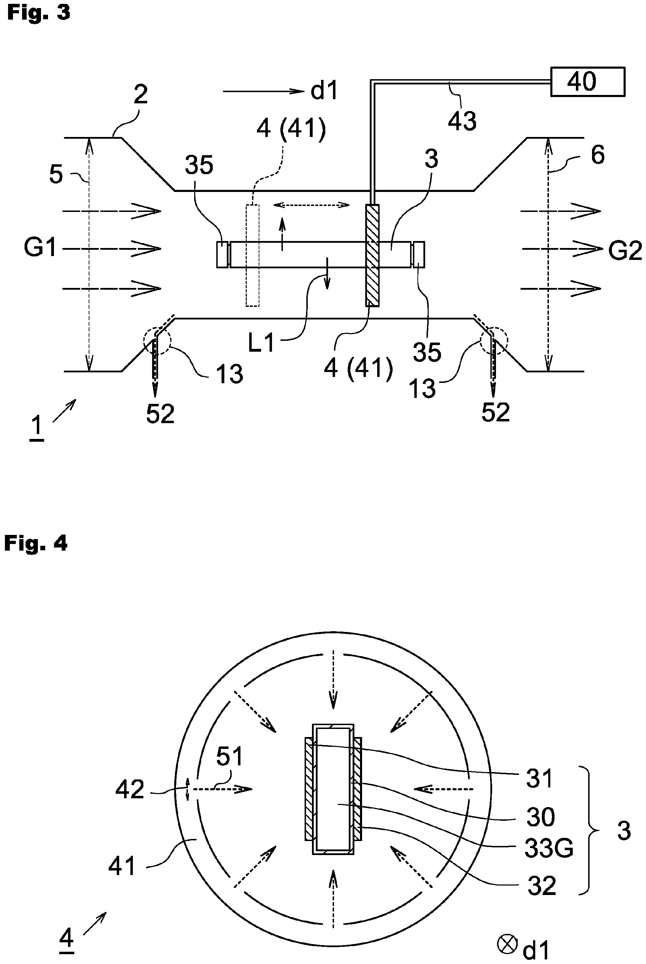

Fig. 3 is a drawing showing, in like manner with Fig. 1, a structure of a gas treatment device 1 according to another embodiment. The gas treatment device of this embodiment differs from the gas treatment device 1 shown in Fig. 1 in configuration of a cleaning mechanism 4. Fig. 4 is a schematic plan view showing the excimer lamp 3 together with the cleaning mechanism 4, viewed along the direction d1.

In the present embodiment, the cleaning mechanism 4, as shown in Fig. 4, includes a nozzle member 41 that has a ring shape and that is disposed so as to substantially surround an outer periphery of the tube body 30 of the excimer lamp 3. The nozzle member 41 has a plurality of holes 42 facing in directions to the tube body 30.

"Substantially surrounding the outer periphery of the tube body 30 of the excimer lamp 3" includes a meaning of surrounding a 50% or greater extent of the outer periphery of the tube body 30, as well as completely surrounding the outer periphery of the tube body 30. In other words, the nozzle member 41 may have an annular shape that lacks a part, such as a C shape or a U shape, as well as a fully annular shape.

Preferably, the cleaning mechanism 4 is allowed to move along the tube body 30 in the direction d1. Fig. 3 also shows the cleaning mechanism 4 indicated by a dashed line and thereby shows that the cleaning mechanism 4 is allowed to move. This configuration allows the cleaning mechanism 4 to spray water as the first fluid 51 over a wide area of the surface of the tube body 30 in the direction d1 and clean the wide area of the surface of the tube body 30. In this case, the cleaning mechanism 4 may be moved manually or may be allowed to move via a drive mechanism (not shown) such as a motor. In the former, a rail designed to move the cleaning mechanism 4 along the direction d1 may be placed inside the housing 2, for example, to allow an operator outside the housing 2 to move the cleaning mechanism 4 along the rail.

The gas treatment device 1 having the configuration shown in Figs. 3 and 4, when a secondary product derived from the VOC is deposited on the surface of the tube body 30, also allows the secondary product to be dissolved in the water acting as the first fluid 51. This can clean the surface of the tube body 30 and prevent a decrease in illuminance of the ultraviolet light L1.

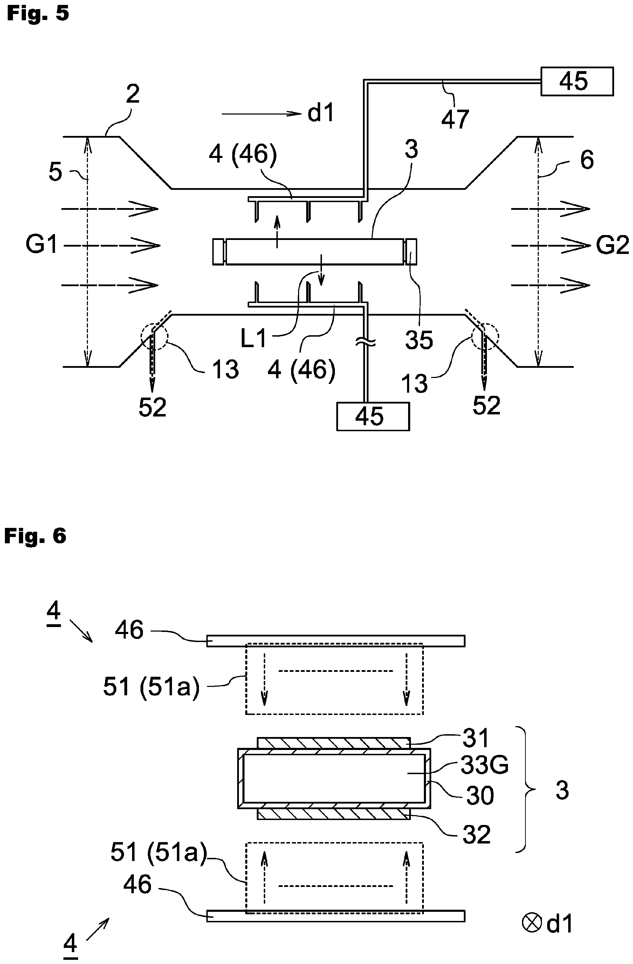

Fig. 5 is a drawing showing, in like manner with Fig. 1, a structure of a gas treatment device 1 according to still another embodiment. The gas treatment device of this embodiment differs from the gas treatment device 1 shown in Fig. 1 in configuration of a cleaning mechanism 4. Fig. 6 is a schematic plan view showing the excimer lamp 3 together with the cleaning mechanism 4, viewed along the direction d1. In the present embodiment, the cleaning mechanism 4, as shown in Fig. 6, includes an air knife 46 to spray humidified air 51a as a first fluid 51 onto the surface of the tube body 30 of the excimer lamp 3.

As shown in Fig. 5, the air knife 46 is supplied with the humidified air 51a as the first fluid 51 from a humidified air source 45 through an air supply pipe 47 and is allowed to splay the humidified air 51a (the first fluid 51) onto the tube body 30. The humidified air 51a is preferably air with 85% or higher relative humidity and is more preferably air with 95% or higher relative humidity.

Fig. 6 shows an instance in which the humidified air 51a ejected as the first fluid 51 from the air knife 46 has a sheet shape, and the sheet-shaped air has an end parallel to a direction of a width of the tube body 30 (a lateral direction of the figure) when viewed along the direction d1. In other words, the present embodiment assumes that the air knife 46, for example, ejects the sheet-shaped humidified air 51a from a plurality of places that are separated from each other in the direction d1. However, the sheet-shaped humidified air 51a may have any shape, and a distal end of the sheet-shaped humidified air 51a may have, for example, a planar shape extending along the direction d1. The air knife 46 may eject the sheet-shaped humidified air 51a acting as the first fluid 51 onto surfaces where the electrodes (31, 32) are not formed, in addition to surfaces where the electrodes (31, 32) are formed, out of outer surfaces of the tube body 30.

The gas treatment device 1 having the configuration shown in Figs. 5 and 6, when a secondary product derived from the VOC is deposited on the surface of the tube body 30, also allows the secondary product to be dissolved in moisture contained in the humidified air 51a. This can clean the surface of the tube body 30 and prevent a decrease in illuminance of the ultraviolet light L1. The gas treatment device 1 is configured to eject the humidified air 51a from the air knife 46 and thereby allows the secondary product to readily come off the surface of the tube body 30 by wind force at the distal end of the sheet-shaped humidified air 51a while allowing the secondary product to be dissolved in the contained moisture.

### (Verification)

As described above in the 'PROBLEMS TO BE SOLVED BY THE INVENTION' section, the inventor treated the gas to be treated G1 using the excimer lamp 3 and found a phenomenon in which dirt (a contaminant) gets deposited on the surface of the tube body 30 of the excimer lamp 3. The inventor checked that letting the gas to be treated G1 flow through inside the housing 2 with the excimer lamp 3 unlit was not enough to cause a contaminant to be deposited on the surface of the tube body 30. Based on the result of this experiment, the inventor presumed that irradiating the gas to be treated G1 with the ultraviolet light L1 emitted from the excimer lamp 3 causes generation of a secondary product and the generated secondary product gets deposited on the surface of the tube body 30.

The inventor presumed that when the gas to be treated G1 containing VOC is irradiated with the ultraviolet light L1 and the VOC is decomposed, a hydroxy group is added and thereby the secondary product apt to be dissolved in water is generated. Thus, the inventor presumed that by causing the cleaning mechanism 4 to spray the first fluid 51 (water, humidified air, or the like) onto the surface of the tube body 30 of the excimer lamp 3 on which the contaminant is deposited, the secondary product could be dissolved and come off the tube body 30, and the inventor conducted an experiment. An outcome of the experiment is described below.

### (Details of experiment)

Before the gas to be treated G1 was introduced into the housing 2, the excimer lamp 3 was lit at 26.4 W, input power of a power source for lighting, and the illuminance at a middle place A of the surface of the tube body 30 in the direction d1 was measured with an illuminometer. The measured illuminance was 30.0 mW/cm².

Next, while the gas to be treated G1 was introduced into the housing 2, the gas to be treated G1 was treated for 300 hours with the excimer lamp 3 lit. Subsequently, the illuminance at the place A was measured with the illuminometer. The measured illuminance was 0.0 mW/cm² (Comparative Example 1). Based on the result of Comparative Example 1, it is inferred that the surface of the tube body 30 of the excimer lamp 3 was covered with a contaminant.

After dry air (10% relative humidity) (Comparative Example 2), water (Example 1), humidified air (85% relative humidity) (Example 2), and humidified air (95% relative humidity) (Example 3) were each sprayed onto the excimer lamp 3 in a situation of Comparative Example 1 for 60 seconds, the illuminances at the place A were measured with the illuminometer. In Example 1, the illuminance at the place A was measured with the illuminometer after drops of the water were removed by blowing ordinary air for 30 seconds following spraying of the water to avoid a state in which the drops of the water remained attached to the surface of the tube body 30. Results yielded in the examples are as shown below in Table 1. In all of Comparative Example 2, Example 2, and Example 3, a flow rate of the air was 37 L/min. In Example 1, the water was supplied at a flow rate of 0.5 L/min.

**[Table 1]**

| | | Illuminance [mW/cm²] |
|---|---|---|
| Before treatment of gas to be treated | | 30.0 |
| After treatment of gas to be treated | Comparative Example 1 (before spraying fluid) | 0.0 |
| | Comparative Example 2 (dry air) | 0.0 |
| | Example 1 (water) | 28.2 |
| | Example 2 (humidified air: 85% relative humidity) | 26.1 |
| | Example 3 (humidified air: 95% relative humidity) | 26.5 |

Comparative Example 2 verifies that even when the dry air was sprayed on the surface of the tube body 30, the illuminance remained unchanged from the condition in Comparative Example 1 and the sprayed air failed to cause the contaminant to come off the surface of the tube body 30. In contrast, in any of Examples 1 to 3, the illuminance increased from that in Comparative Example 1 and degree of decline from the illuminance before introduction of the gas to be treated G1 into the housing 2 was very small compared to that in Comparative Examples 1 to 2. This proves that spraying water or humidified air onto the surface of the tube body 30 allows the contaminant to be dissolved in the water and come off the surface of the tube body 30.

It is deduced that a reason why the method in Comparative Example 2 failed to cause the contaminant to come off the surface of the tube body 30 is, after the VOC-derived secondary product turns into aerosols and comes into contact with the surface of the tube body 30, the aerosols are polymerized and the contaminant thereby having viscosity gets deposited firmly on the surface of the tube body 30. Meanwhile, in a course of decomposition of the VOC, a reaction by which a hydroxy group is added occurs. Presumably, the secondary product contains the hydroxy group and hence contains a substance apt to be dissolved in water. Actually, while the gas to be treated G1 that is a gas containing xylene as VOC was introduced into the housing 2, the gas to be treated G1 was treated with the excimer lamp 3 lit. The inventor analyzed a secondary product deposited on the tube body 30 of the excimer lamp 3 and verified that 2,5/2,6-xylenol, 2,5/2,6-dimethyl hydroquinone, and 4,5-dimethylresorcinol were contained. All these are substances containing hydroxy groups.

### [Other embodiments] Other embodiments will now be described.

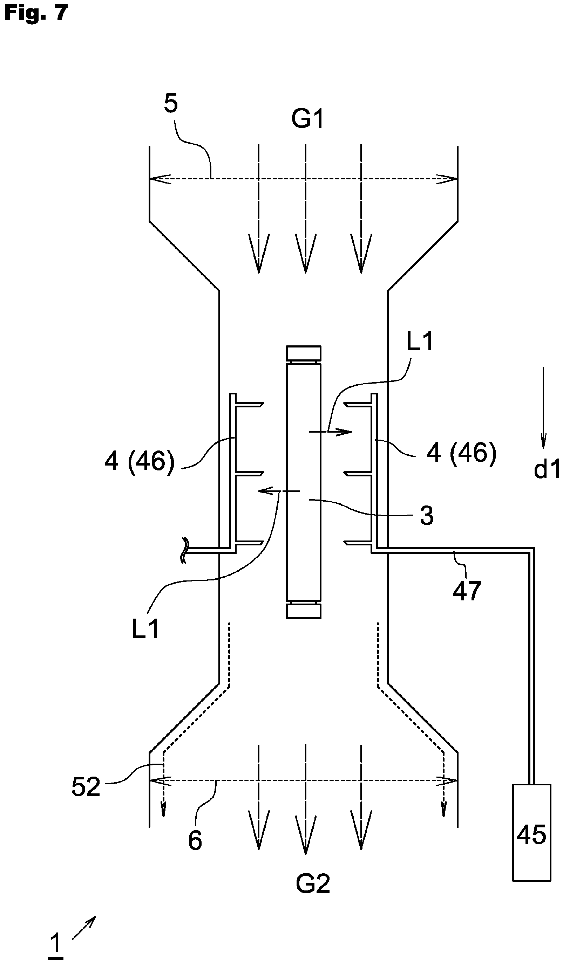

<1 > The gas treatment device 1 may be disposed in any direction. In one example, as shown in Fig. 7, the gas treatment device 1 may be disposed in the direction d1 specified as a vertical direction. In this case, the fluid (the second fluid 52), which is produced when the VOC-derived secondary product deposited on the surface of the tube body 30 is dissolved in the water, moves in the housing 2 downward by gravity. Thus, as shown in Fig. 7, the gas treatment device 1 may not necessarily include the drainage outlet 13. In a configuration shown in Fig. 7, the gas treatment device 1 may include a reservoir (not shown) to temporarily store the second fluid 52, which runs along an inner wall surface of the housing 2 and flows to outside the housing 2 from the gas outlet 6.

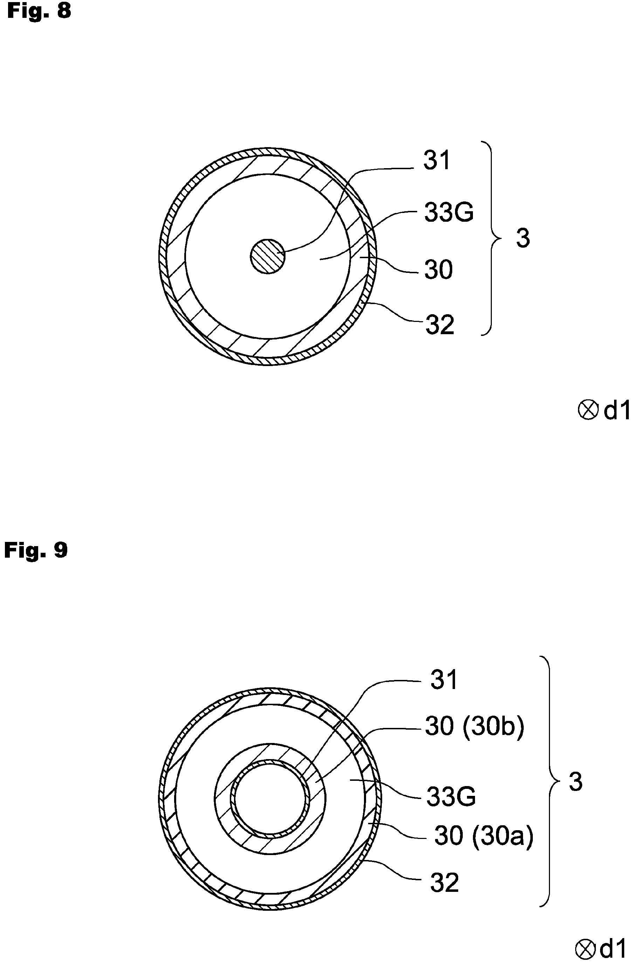

<2> In the present invention, the excimer lamp 3 may have any structure. For instance, as shown in Fig. 8, the excimer lamp 3 may have what is called a "single-tube structure", or as shown in Fig. 9, the excimer lamp 3 may have what is called a "double-tube structure". Figs. 8 and 9 are schematic views each showing a structure of the excimer lamp 3, viewed along the direction d1.

The excimer lamp 3 shown in Fig. 8 includes the first electrode 31 disposed inside the tube body 30, the discharge gas 33G filling the interior of the tube body 30, and the second electrode 32 disposed on an outer wall surface of the tube body 30. In this case, the first electrode 31 extends along the direction d1 in shape and the second electrode 32 has a mesh shape or a linear shape.

The excimer lamp 3 shown in Fig. 9 has two tube bodies 30 (30a, 30b). The excimer lamp includes the tube body 30b, which is cylindrical and is disposed on an external side, and the tube body 30a, which is cylindrical and is disposed inside the tube body 30b so as to be coaxial with the tube body 30b. An inner diameter of the tube body 30a is smaller than that of the tube body 30b. The tube body 30a and the tube body 30b are sealed off at each end (not shown) in the direction d1. An annular light-emission space is formed between the two tube bodies. The space is filled with the discharge gas 33G. The first electrode 31 is disposed on an inner wall surface of the inside tube body 30a, and the second electrode 32 is disposed on an outer wall surface of the outside tube body 30b. The first electrode 31 has a layer shape, and the second electrode 32 has a mesh shape or a linear shape.

<3> The configurations of the cleaning mechanism 4 in the embodiments described above are merely examples. The cleaning mechanism 4 included in the gas treatment device 1 of the present invention is not limited to the illustrated forms. In other words, the cleaning mechanism 4 may have any configuration with the proviso that the cleaning mechanism is allowed to spray the first fluid 51 containing moisture from an inside of the housing 2 onto the surface of the tube body 30. The cleaning mechanism 4 may be detachable from the gas treatment device 1.

In the above embodiments, cases are described in which the first fluid 51 is either of water or humidified air. However, in the former, i.e., in the case in which the first fluid 51 is water, the first fluid 51 is not necessarily pure water. For instance, the first fluid 51 may be water containing a surface-active agent. This enhances the effect of causing the secondary product deposited on the surface of the tube body 30 to come off. The first fluid 51 may be water containing any of other substances except VOC, as well as the surface-active agent. Moreover, when the first fluid 51 is water or water containing any of the other substances, the first fluid may not be a pure liquid but may be made up of a mist of drops with a particle diameter ranging approximately from several micrometers to several tens of micrometers or a mixture of the mist and air. In other words, in the present invention, the first fluid 51 conceptually includes a case in which a fluid is in the form of minute particles floating in a gas (or being atomized).

In the latter, i.e., in the case in which the first fluid 51 is humidified air, the first fluid 51 is not necessarily a gas of pure air that is humidified. The first fluid may be a gas of humidified air containing any of other substances except VOC-derived substances.

### DESCRIPTION OF REFERENCE SIGNS

- 1: Gas treatment device
- 2: Housing
- 3: Excimer lamp
- 4: Cleaning mechanism
- 5: Gas inlet
- 6: Gas outlet
- 13: Drainage outlet
- 30: Tube body
- 30a: Tube body
- 30b: Tube body
- 31: First electrode
- 32: Second electrode
- 33G: Discharge gas
- 35: Base
- 40: Water supply source
- 41: Nozzle member
- 42: Hole
- 43: Water supply pipe
- 45: Humidified air source
- 46: Air knife
- 47: Air supply pipe
- 48: Water injection pipe
- 51: First fluid
- 51a: Humidified air
- 52: Second fluid
- G1: Gas to be treated
- G2: Treated gas
- L1: Ultraviolet light
- d1: Direction

## Claims

1. A gas treatment device, comprising:
a housing;
a gas inlet to introduce a gas to be treated containing volatile organic compounds (VOC) into the housing;
an excimer lamp housed inside the housing, the excimer lamp including a tube body with a long shape extending in a first direction, an interior of the tube body being filled with a discharge gas;
a gas outlet to exhaust the gas to be treated to outside the housing, the gas to be treated being irradiated with ultraviolet light emitted from the excimer lamp; and
a cleaning mechanism to spray a first fluid from an inside of the housing onto a surface of the tube body, the first fluid containing moisture and being used for cleaning.

2. The gas treatment device according to claim 1, wherein
the excimer lamp includes a base made of an inorganic material at an end of the tube body in the first direction, and
the cleaning mechanism sprays the first fluid onto the surface of the tube body positioned inward from the base.

3. The gas treatment device according to claim 1 or 2, wherein the housing includes a drainage outlet to drain a second fluid in liquid form to outside the housing, the second fluid being left after the surface of the tube body is cleaned with the first fluid.

4. The gas treatment device according to any one of claims 1 to 3, wherein
the cleaning mechanism includes:
a water injection pipe disposed so as to pass through a part of the housing; and a water supply pipe disposed outside the housing to supply water as the first fluid to the water injection pipe, and
the water injection pipe sprays the water as the first fluid onto the surface of the tube body.

5. The gas treatment device according to any one of claims 1 to 3, wherein
the cleaning mechanism includes:
a nozzle member that has a ring shape so as to substantially surround an outer periphery of the surface of the tube body and that has a plurality of holes on a surface facing the tube body; and
a water supply pipe to supply water as the first fluid to the nozzle member.

6. The gas treatment device according to claim 5, wherein the cleaning mechanism allows a target onto which the first fluid is sprayed to be shifted along the first direction.

7. The gas treatment device according to any one of claims 1 to 3, wherein the cleaning mechanism includes an air knife to spray humidified air as the first fluid in planar form.

8. The gas treatment device according to any one of claims 1 to 7, wherein the excimer lamp includes a first electrode disposed at a predetermined place on an outer surface of the tube body and a second electrode that is disposed on an outer surface of the tube body and at a place opposite to the first electrode through the interior of the tube body filled with the discharge gas.

9. The gas treatment device according to any one of claims 1 to 7, wherein the excimer lamp includes a first electrode disposed at a predetermined place on an outer surface of the tube body and a second electrode disposed in the interior of the tube body filled with the discharge gas.

10. The gas treatment device according to any one of claims 1 to 7, wherein
the excimer lamp has a double-tube structure such that the tube body includes an outside tube and an inside tube disposed inside the outside tube and such that the outside tube and the inside tube are sealed off at both ends of the tube body in the first direction,
a space between the inside tube and the outside tube is filled with the discharge gas, and
the excimer lamp includes a first electrode disposed on an outer surface of the outside tube and a second electrode disposed on an inner surface of the inside tube.
